(19) Europäisches Patentamt
European Patent Office
Office européen des brevets

(11) Publication number: 0 171 853 B1

(12) **EUROPEAN PATENT SPECIFICATION**

(45) Date of publication of patent specification: 22.03.89

(51) Int. Cl.$^4$: C 07 C 161/00, A 01 N 47/34, C 07 C 154/00

(21) Application number: 85201258.2

(22) Date of filing: 31.07.85

(54) **Pesticidal benzoylurea compounds.**

(30) Priority: 17.08.84 GB 8420930

(43) Date of publication of application: 19.02.86 Bulletin 86/08

(45) Publication of the grant of the patent: 22.03.89 Bulletin 89/12

(84) Designated Contracting States: AT BE CH DE FR GB IT LI LU NL SE

(56) References cited: EP-A-0 103 918

(73) Proprietor: SHELL INTERNATIONALE RESEARCH MAATSCHAPPIJ B.V.
Carel van Bylandtlaan 30
NL-2596 HR Den Haag (NL)

(72) Inventor: Anderson, Martin
92 Clare Road
Whitstable Kent (GB)

(74) Representative: Hunter, Keith Roger Ian et al
4 York Road
London SE1 7NA (GB)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 171 853 B1

## Description

The present invention relates to benzoylurea compounds having pesticidal, especially insecticidal and acaricidal, activity.

UK Patent Specification No. 1,324,293 discloses a class of urea derivatives having insecticidal activity. These compounds are highly active, and include the commercial compound, N-(2,6-difluorobenzoyl)-N'-(4-chlorophenyl)urea.

EP—A—103 918 discloses benzoylurea compounds of formula

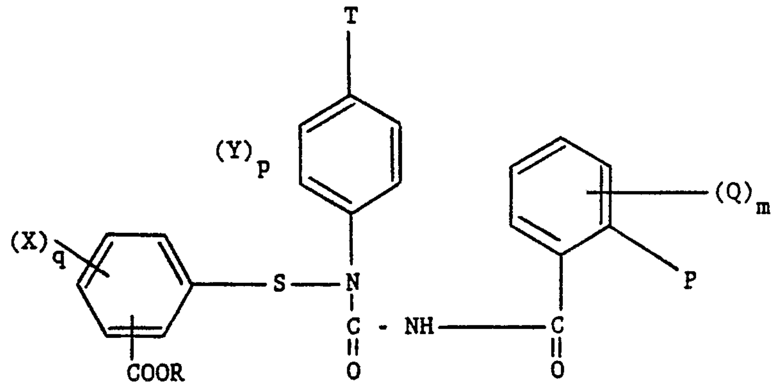

in which each of P and Q independently represents a halogen atom or an alkyl group; m represents 0, 1 or 2; R represents an optionally-substituted alkyl, alkenyl, alkynyl, cycloalkyl or phenyl group, a hydrogen atom, one equivalent of an alkali metal or alkaline earth metal, or an ammonium or substituted ammonium group; each X independently represents a halogen atom, a cyano, nitro or carboxy group, or an optionally-substituted alkyl, alkoxy, alkylthio, cycloalkyl, cycloalkyloxy, cycloalkylthio, alkenyl, alkenylthio, alkylcarbonyl, alkoxycarbonyl, alkylsulphonyl, alkynyl, phenyl, phenoxy, phenylthio or amino group; q = 0, 1, 2, 3 or 4; T represents a halogen atom, an alkyl, haloalkyl, alkoxy, haloalkoxy, alkenyloxy or haloalkenyloxy group, or a phenoxy or pyridyloxy group optionally substituted by one or more substituents selected from halogen atoms and nitro, alkyl, haloalkyl and cyano groups; Y independently represents a halogen atom or a nitro, cyano, alkyl or haloalkyl group; and p = 1, 2, 3 or 4; and the pesticidal activity of these compounds.

The Applicants have now discovered a novel class of urea-type compounds which not only have high insecticidal activity, but also possess acaricidal activity.

The invention provides a compound of the general formula

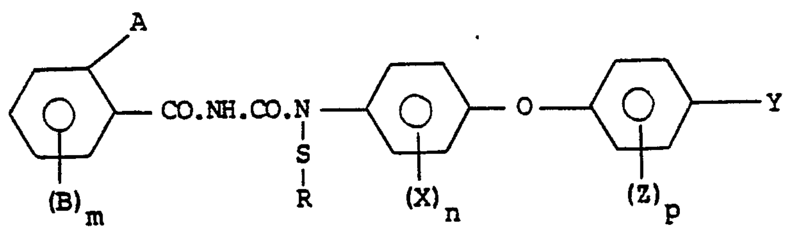

(I)

in which A and B independently represents a halogen atom or an alkyl group; m is 0, 1 or 2; X, Y and Z independently represents a halogen atom or a cyano, nitro, alkyl or haloalkyl group, provided that any X present is not a fluorine atom ortho to the nitrogen atom; n is 0, 1, 2, 3 or 4; p is 0, 1 or 2; and R represents a group $-CO_2R^1$, $-SO_2R^1$ or $-NR^2R^3$, in which $R^1$ represents an optionally substituted alkyl or aryl group; $R_2$ represents an optionally substituted alkyl or aryl group; and $R_3$ represents an optionally substituted alkyl or aryl group, or a group of formula $-CO_2R^4$, $-SO_2R^4$, $-COR^4$, $-CO.CO_2R^4$, $-CO.NR^5R^6$ or $-SO_2NR^5R^6$, in which $R^4$ represents an optionally substituted alkyl or aryl group, and each of $R^5$ and $R^6$ independently represents an optionally substituted alkyl or aryl group; or $R^2$ and $R^3$ together or $R^5$ and $R^6$ together represent an optionally substituted alkylene group; in each case, the optional substituents for an alkyl or alkylene group being selected from halogen, alkoxy, alkoxycarbonyl, haloalkoxycarbonyl, alkylcarbonyl, haloalkylcarbonyl, alkylsulphonyl and haloalkylsulphonyl, and the optional substituents for an aryl group being selected from these substituents and also alkyl, haloalkyl, cyano and nitro.

Except where otherwise stated, throughout this Specification and claims, any aryl group is preferably a phenyl group. When $R^1$, $R^2$, $R^3$, $R^4$, $R^5$ or $R^6$ represents an optionally substituted alkyl group, the alkyl moiety preferably has up to 12, especially up to 6, carbon atoms. Any other alkyl moiety present in the compound of the general formula I preferably has up to 6, especially up to 4, carbon atoms, a preferred alkyl group

being methyl, and a preferred haloalkyl group being trifluoromethyl. Any alkylene moiety preferably has 4 to 8 carbon atoms, and is preferably a tetramethylene or pentamethylene group. Halogen atoms may be fluorine, chlorine, bromine or iodine atoms, with fluorine and chlorine being preferred. When m, n or p is greater than 1, the substituents B, X or Z, respectively, may be the same or different.

Preferably each of A and B independently represents a fluorine or chlorine atom or a methyl group. Preferably m is 0 or 1. Most preferably, A is a fluorine atom, m is 1 and B is a fluorine or chlorine atom, preferably in the 6-position of the phenyl ring (A being in the 2-position).

X may represent, for example, a chlorine atom or a methyl group or a fluorine atom meta to the nitrogen atom. Thus typically $(X)_n$ may be 1 or 2 fluorine atoms and a chlorine atom, or 1 or 2 fluorine atoms and two chlorine atoms, each fluorine atom being meta to the nitrogen atom. Most preferably each X represents a fluorine or chlorine atom and n is 2, or especially, n is 1 or 0.

Preferably Y represents a chlorine atom or a nitro, cyano or trifluoromethyl group. A trifluoromethyl group Y is especially preferred.

Preferably Z represents a chlorine atom or a cyano or nitro group. Preferably p is 0 or 1. Most preferably Z represents a chlorine atom and p is 1; such a chlorine atom is preferably in the position meta to Y.

Preferably each of $R^1$, $R^2$, $R^4$, $R^5$ and $R^6$, when present, represents an unsubstituted alkyl group having up to 6 carbon atoms.

Preferably R represents a group of formula $—NR^2R^3$. Preferably $R^3$ represents an alkyl group having up to 6 atoms substituted by an alkoxycarbonyl group having up to 6 carbon atoms in the alkyl moiety, or a group of formula $—CO_2R^4$, $—SO_2R^4$, $—COR^4$, $—CO.CO_2R^4$, $—CO.NR^5R^6$ or $—SO_2NR^5R^6$ .

Especially preferred groups R are those of formula $—NR^2CO_2R^4$ in which each $R^2$ nd $R^4$ represents an unsubstituted alkyl group having up to 6 carbon atoms.

The invention also provides a process for the preparation of a compound of the general formula I, which comprises reacting a compound of the general formula

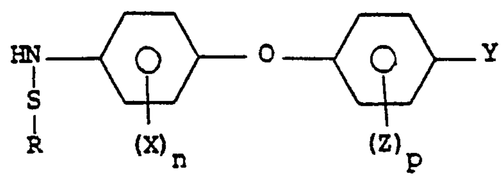

(II)

with a compound of the general formula

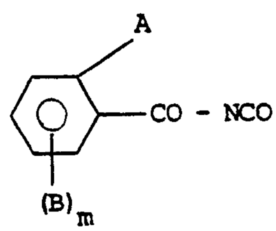

(III)

in which A, B, m, R, X, Y, Z, n and p have the meanings given for the general formula I.

The reaction is suitably carried out in the presence of a solvent. Suitable solvents are aromatic solvents such as benzene, toluene, xylene, or chlorobenzene, hydrocarbons such as petroleum fractions, chlorinated hydrocarbons such as chloroform, methylene chloride or dichloroethane, and ethers such as diethylether, dibutylether, or dioxan. Mixtures of solvents are also suitable.

Preferably the reaction is carried out at temperature from 0°C to 100°C, suitably ambient temperature. Preferably the molar ratio of isocyanate to amine is from 1:1 to 2:1. Preferably the reaction is carried out under anhydrous conditions.

The compounds of formula II are themselves novel and constitute a further aspect of the invention. They may be prepared by reacting a compound of the general formula

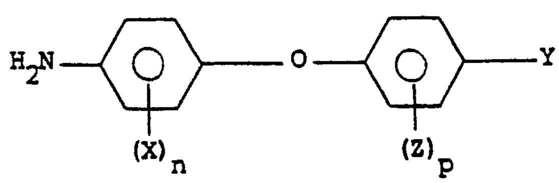

(IV)

with a compound of the general formula

Hal—S—R (V)

in which X, Y, Z, n, p and R have the meanings given above, and Hal represents a halogen, especially chlorine, atom. The reaction is preferably carried out in the presence of an inert solvent, for example a hydrocarbon or chlorinated hydrocarbon, and the reaction temperature is preferably in the range of from −30 to +30°C, preferably −10 to +10 °C. The reaction is suitably carried out in the presence of a base, for example an amine such as triethylamine.

The compounds of the general formula I exhibit pesticidal, for example insecticidal and acaricidal, activity. Accordingly the invention also provides a pesticidal composition comprising a compound of the general formula I together with a carrier. The invention further provides a method of combating pests at a locus, which comprises applying to the locus a pesticidal compound or composition according to the invention. The pests may be insects or acarids, especially mites. The locus may be a crop area susceptible to infestation by acarids.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating pesticidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3—10% w of a dispersing agent and, where necessary, 0—10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by agglomeration or impregnation techniques. Generally, granules will contain ½—75% w active ingredient and 0—10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the

formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil type or of the oil-in-water type, and may have a thick 'mayonnaise'-like consistency.

The composition of the invention may also contain other ingredients, for example other compounds possessing pesticidal, herbicidal, or fungicidal properties. The compounds of the invention are especially useful when applied in admixture with other insecticides, especially organophosphates and pyrethroids. Mixtures with the commercial products fenvalerate, permethrin, cypermethrin, deltamethrin and alphamethrin are especially useful.

The following Examples illustrate the invention; Examples 1 to 3 illustrate the preparation of intermediates, while Examples 4 to 6 illustrate the preparation of compounds of the formula I.

## Example 1

*Preparation of propyl N-[[[4-[2-chloro-4-(trifluoromethyl)phenoxy]phenyl]amino]thio]-N-methylcarbamate*

A solution of dry N-chlorosulphenyl-N-methylcarbamate (3.7g) in dry methylene chloride (10ml) was added over 20 minutes to a stirred solution of 4-[2-chloro-4-(trifluoromethyl)phenoxy]aniline (6.3g) in the same solvent (25ml) containing triethylamine (2.5g). The reaction temperature was kept at 0—5°C by means of an ice bath until the addition was completed, and was then allowed to rise to ambient temperature over 2.0 hours. The solvent was removed under reduced pressure and the residue was suspended in diethyl ether (200ml) and washed three times with water. The resulting ether solution was dried over magnesium sulphate and evaporated to yield the crude product which was purified by rapid chromatography on silica gel using methylene chloride as eluent. Crystallisation from diethyl ether/light petroleum afforded the pure product as pale buff-coloured crystals (7.1g) melting at 61—64°C.

The following analytical results were obtained:

| | | | |
|---|---|---|---|
| Calculated: | C; 49.7% | H; 4.6% | N; 6.4% |
| Found | C; 49.4% | H; 4.2% | N; 6.3% |

## Example 2

*Preparation of N-[[[4-[2-chloro-4-(trifluoromethyl)phenoxy]phenyl]amino]thio]-N-ethylmethanesulphonamide*

A solution of sulphur dichloride (2.6g) in dry diethyl ether (15ml) was added over 10 minutes to a stirred solution of N-ethylmethanesulphonamide (3.1g) in the same solvent (15ml) keeping the temperature between 10—15° by means of external cooling. A solution of dry pyridine (2.0g) in dry diethyl ether (15ml) was then added over 15 minutes at the same temperature and the reaction mixture was allowed to warm to room temperature over 1 hour. The resulting suspension was transferred to a dropping funnel and added over 5 minutes to a solution of 4-[2-chloro-4-(trifluoromethyl)phenoxy]aniline (7.2g) in dry diethyl ether (75ml) containing pyridine (2.0g) keeping the temperature between 5—10°C. After stirring at 10—15° for one hour, the reaction mixture was washed three times with water, and the ether layer was separated and dried over magnesium sulphate. Evaporation of the dried solution under reduced pressure afforded the crude product which was purified by chromatography on silica gel using diethyl ether as eluent. The pure product was thus obtained as a buff solid (6.9g) melting at 98—101°C.

The following analytical results were obtained:

| | | | |
|---|---|---|---|
| Calculated: | C; 43.6% | H; 3.6% | N; 6.4% |
| Found | C; 43.88% | H; 3.8% | N; 6.2% |

## Example 3

*Preparation of O-Methyl-S-[[4-[2-chloro-4-(trifluoromethyl)phenoxy]phenyl]amino]thiocarbonate*

A solution of methoxycarbonylsulphenyl chloride (4.2 g) in dry methylene chloride (5 ml) was added over 20 minutes to a stirred solution of 4-[2-chloro-4-(trifluoromethyl)phenoxy]aniline (8.6 g) in the same solvent (50 ml) containing triethylamine (6.0 ml) keeping the temperature below 0°C. After the addition was complete the stirred reaction mixture was allowed to warm to room temperature over 1.0 hour. The solvent was then removed under reduced pressure and the residue was suspended in diethyl ether (180 ml) and washed with water. Evaporation of the dried extract gave 11.5 g of a brown oil which was purified by chromatography on silica gel using diethyl ether/petroleum ether as eluent. Recrystallisation from the same solvent yielded 7.0 g of the desired product as colourless crystals, melting point 71—72°C.

The following analytical results were obtained:

| | | | |
|---|---|---|---|
| Calculated: | C; 47.7% | H; 2.9% | N; 3.7% |
| Found: | C; 48.2% | H; 3.0% | N; 3.8% |

Example 4

*Preparation of propyl 4-[4-[2-chloro-4-(trifluoromethyl)phenoxy]phenyl]-7-(2,6-difluorophenyl)-2-methyl-5,7-dioxo-3-thia-2,4,6-triazaheptanoate*

A solution of 2,6-difluorobenzoyl isocyanate (2.4 g) in dry toluene (10 ml) was added, with stirring, to a solution of propyl N-[[[4-[2-chloro-4-(trifluoromethyl)phenoxy]phenylamino]thio]-N-methylcarbamate (5.2 g) in the same solvent (40 ml) and the resulting solution was stirred at room temperature for 3 hours. The reaction mixture was then diluted with dry light petroleum (bp. 40—60°C, 50 ml) and stored at −5—0°C overnight.

The resulting precipitate of product was filtered off, washing with light petroleum. Recrystallisation from diethyl ether/light petroleum afforded the pure product as colourless crystals (5.8 g), melting at 96—98°C.

The following analytical results were obtained:

| | | | |
|---|---|---|---|
| Calculated: | C; 50.5% | H; 3.4% | N; 6.8% |
| Found: | C; 51.2% | H; 3.7% | N; 6.6% |

Example 5

*Preparation of N-[[[4-(2-chloro-4-(trifluoromethyl)phenoxy]phenyl][[(N-ethyl-N-methanesulfonyl)amino]-thio]amino]carbonyl]-2,6-difluorobenzamide*

A solution of 2,6-difluorobenzoyl isocyanate (2.0 g) in dry diethyl ether (10 ml) was added rapidly to a stirred solution of N-[[[4-[2-chloro-4-(trifluoromethyl)phenoxy]phenyl]amino]thio]-N-ethylmethane-sulfonamide (4.4 g) in the same solvent (15 ml) at room temperature. After stirring for 4 hours, light petroleum (bp 40—60°C, 10 ml) was added and the resulting precipitate of product was filtered off, washing with diethyl ether/light petroleum. Recrystallisation of this material from diethyl ether/light petroleum afforded the pure product as colourless crystals (5.8 g), melting at 149—151°C.

The following analytical results were obtained:

| | | | |
|---|---|---|---|
| Calculated: | C; 46.2% | H; 3.1% | N; 6.7% |
| Found: | C; 46.7% | H; 3.0% | N; 6.7% |

Example 6

*Preparation of Methyl-3-[4-[2-chloro-4-(trifluoromethyl)phenoxy]phenyl]-6-(2,6-difluorophenyl)-4,6-dioxo-2-thia-3,5-diazahexanoate*

A solution of 2,6-difluorobenzoyl isocyanate (2.0 g) in dry toluene (10 ml) was added to a stirred solution of O-methyl-S-[[4-[2-chloro-4-(trifluoromethyl)-phenoxy]phenyl]amino]thiocarbonate (3.8 g) in the same solvent (30 ml). The mixture was stirred at room temperature for 3 hours. It was then diluted with an equal volume of petroleum ether and cooled in ice-water. After 1 hour, the mixture was cooled to −70°C for 10 minutes, and the resulting crop of crystals was filtered off, washed with petroleum ether, and dried. Recrystallisation from petroleum ether gave 5.3 g of the desired product, melting point 108—109°C.

The following analytical results were obtained:

| | | | |
|---|---|---|---|
| Calculated: | C; 49.3 | H; 2.5 | N; 5.0 |
| Found: | C; 49.5 | H; 2.5 | N; 4.9% |

Examples 7 to 18

By methods analogous to these described in the previous Examples, further compounds of the general formula I were prepared. Details are given in Table I.

TABLE I

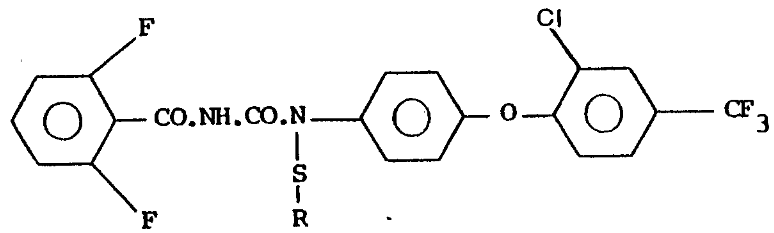

| Example No | In the Formula Above R | Melting Point °C | | Elemental Analysis % C | H | N |
|---|---|---|---|---|---|---|
| 7 | $N(CH_3)CO_2CH_3$ | 96-100 | Calculated | 48.9 | 2.9 | 7.1 |
| | | | Found | 49.0 | 3.0 | 6.9 |
| 8 | $N(CH_3)CO_2nC_5H_{11}$ | 69-71 | Calculated | 52.1 | 3.8 | 6.5 |
| | | | Found | 52.6 | 3.9 | 6.4 |
| 9 | $N(C_2H_5)CO_2C_2H_5$ | 82-85 | Calculated | 50.5 | 3.4 | 6.8 |
| | | | Found | 50.9 | 3.7 | 6.5 |

Table I (continued)

| Example No | In the Formula Above R | Melting Point °C | | Elemental Analysis % C | H | N |
|---|---|---|---|---|---|---|
| 10 | $N(tC_4H_9)CO_2CH_3$ | 82-85 | Calculated | 51.3 | 3.6 | 6.7 |
| | | | Found | 51.7 | 4.1 | 6.3 |
| 11 | $N(tC_4H_9)CO_2C_2H_5$ | 110-113 | Calculated | 52.1 | 3.9 | 6.5 |
| | | | Found | 52.7 | 4.1 | 6.3 |
| 12 | $N(nC_4H_9)SO_2CH_3$ | 142-144 | Calculated | 47.9 | 3.5 | 6.5 |
| | | | Found | 47.6 | 3.4 | 6.2 |
| 13 | $CO_2nC_3H_7$ | 94-95 | Calculated | 51.0 | 3.1 | 4.8 |
| | | | Found | 51.2 | 3.0 | 4.7 |
| 14 | $CO_2nC_5H_7$ | 82-83 | Calculated | 52.6 | 3.6 | 4.5 |
| | | | Found | 53.1 | 3.6 | 4.5 |

Table I (continued)

| Example No | In the Formula Above R | Melting Point °C | | Elemental Analysis % C | H | N |
|---|---|---|---|---|---|---|
| 15 | $N(nC_4H_9)CO_2CH_3$ | 109-112 | Calculated | 51.3 | 3.6 | 6.7 |
| | | | Found | 51.5 | 3.6 | 6.5 |
| 16 | $N(tC_4H_9)CO_2tC_4H_9$ | 80-82 | Calculated | 53.5 | 4.3 | 6.2 |
| | | | Found | 53.5 | 4.2 | 5.9 |
| 17 | $N(CH_3)CO_2iC_3H_7$ | 95-98 | Calculated | 50.5 | 3.4 | 6.8 |
| | | | Found | 51.2 | 3.5 | 6.5 |
| 18 | $N(CH_3)CO_2tC_4H_9$ | 122-124 | Calculated | 51.3 | 3.6 | 6.5 |
| | | Found | 51.4 | 3.5 | 6.2 | |

Examples 19 to 30

By methods analogous to those described in Examples 1 to 3, further intermediates of the general formula II were prepared. Details are given in Table II.

TABLE II

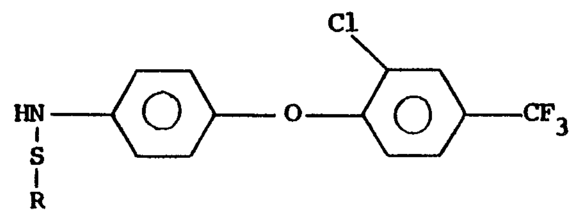

| Example No | In the Formula Above R | Melting Point °C | | Elemental Analysis % C | H | N |
|---|---|---|---|---|---|---|
| 19 | $N(CH_3)CO_2CH_3$ | 106-109 | Calculated | 47.2 | 3.4 | 6.9 |
| | | | Found | 47.5 | 3.7 | 6.8 |
| 20 | $N(CH_3)CO_2nC_5H_{11}$ | 74-76 | Calculated | 51.9 | 4.8 | 6.1 |
| | | | Found | 51.9 | 5.2 | 6.0 |
| 21 | $N(C_2H_5)CO_2C_2H_5$ | 76-78 | Calculated | 49.7 | 4.1 | 6.4 |
| | | | Found | 49.7 | 4.4 | 6.4 |
| 22 | $N(tC_4H_9)CO_2CH_3$ | Not Isolated | | | | |

Table II (continued)

| Example No | In the Formula Above R | Melting Point °C | | Elemental Analysis % C | H | N |
|---|---|---|---|---|---|---|
| 23 | $N(tC_4H_9)CO_2C_2H_5$ | | Not Isolated | | | |
| 24 | $N(nC_4H_9)SO_2CH_3$ | 97-98 | Calculated | 46.1 | 4.3 | 6.0 |
| | | | Found | 46.2 | 4.4 | 5.9 |
| 25 | $CO_2nC_3H_7$ | 61-62 | Calculated | 50.3 | 3.7 | 3.5 |
| | | | Found | 50.7 | 3.7 | 3.5 |
| 26 | $CO_2nC_5H_7$ | 54-55 | Calculated | 52.6 | 4.4 | 3.2 |
| | | | Found | 52.8 | 4.3 | 3.1 |
| 27 | $N(nC_4H_9)CO_2CH_3$ | | Not Isolated | | | |
| 28 | $N(tC_4H_9)CO_2tC_4H_9$ | | Not Isolated | | | |

Table II (continued)

| Example No | In the Formula Above R | Melting Point °C | Elemental Analysis % C | H | N |
|---|---|---|---|---|---|
| 29 | $N(CH_3)CO_2iC_3H_7$ | | Not Isolated | | |
| 30 | $N(CH_3)CO_2tC_4H_9$ | | Not Isolated | | |

Example 31

*Insecticidal Activity*

The insecticidal activity of the compounds of the invention was determined in the following tests, employing the pests *Spodoptera littoralis* (S.l.) and *Aedes aegypti* (A.a.).

The test methods used for each species appear below. In each case the tests were conducted under normal conditions (23°C ± 2°C; fluctuating light and humidity).

In each test an $LC_{50}$ (the dosage of active material required to kill half of the test species) for the compound was calculated from the mortality figures and compared with the corresponding $LC_{50}$ for a standard insecticide, ethyl parathion, in the same tests. The results are expressed as toxicity indices thus:

$$\text{toxicity index} = \frac{LC_{50}\ \text{(parathion)}}{LC_{50}\ \text{(test compound)}} \times 100$$

and are set out in Table III below.

(i) *Spodoptera littoralis*

Solutions or suspensions of the compound were made up over a range of concentrations in 10% acetone/water containing .025% Triton X100 ("Triton" is a registered trade mark). These solutions were sprayed using a logarithmic spraying machine onto petri dishes containing a nutritious diet on which the *Spodoptera littoralis* larvae had been reared. When the spray deposit had dried each dish was infested with 10 2nd instar larvae. Mortality assessments were made 7 days after spraying.

(ii) *Aedes aegypti*

Several solutions of the test compound of varying concentration were prepared in acetone. 100 microlitre quantities were added to 100 ml of tap water, the acetone being allowed to evaporate off. 10 early 4th instar larvae were placed in the test solution; after 48 hours the (surviving) larvae were fed with animal feed pellets, and the final percentage mortality assessed when all the larvae had either pupated and emerged as adults or died.

TABLE III
Insecticidal Activity

| Compound of Example No. | S.l. | A.a. |
|---|---|---|
| 4 | 2900 | 600 |
| 5 | 3900 | 850 |
| 6 | 1100 | 190 |
| 7 | 4100 | 990 |
| 8 | 4000 | 860 |
| 9 | 4100 | 680 |
| 10 | 3700 | 630 |
| 11 | 2400 | 650 |
| 12 | 3900 | 800 |
| 13 | 2000 | 310 |
| 14 | 2100 | 120 |
| 15 | 5800 | 740 |
| 16 | 7300 | 820 |
| 17 | 4200 | 930 |
| 18 | 3400 | 850 |

Example 32

*Acaricidal Activity*

Leaf discs were infested with 30—60 larvae of the mite *Tetranicus urticae* and sprayed with varying dosages of solutions of the test compound made up as in test (i) of Example 31 above. When dry, the discs were maintained at constant temperature for 12 days, after which mortality assessments were made, and the $LC_{50}$ values calculated. Also included in the test was the commercial compound N-(2,6-difluorobenzoyl)-N'-(4-chlorophenyl)urea, of UK Specification 1,324,293. The results show that the compounds of the present invention exhibit considerably greater activity than the commercial compound.

TABLE IV
Acaricidal Activity

| Compound of Example No. | $LC_{50}$ (% active ingredient in spray) |
|---|---|
| 4 | 0.00027 |
| 5 | 0.00020 |
| 6 | 0.0010 |
| 7 | 0.00018 |
| 8 | 0.00017 |
| 9 | 0.00027 |
| 10 | 0.00022 |
| 11 | 0.00032 |
| 12 | 0.00015 |
| 13 | 0.0030 |
| 14 | 0.00073 |
| 15 | 0.00016 |
| 16 | 0.00014 |
| 17 | 0.000096 |
| 18 | 0.00014 |
| Comparison compound | >0.1% |

**Claims**

1. A compound of the general formula

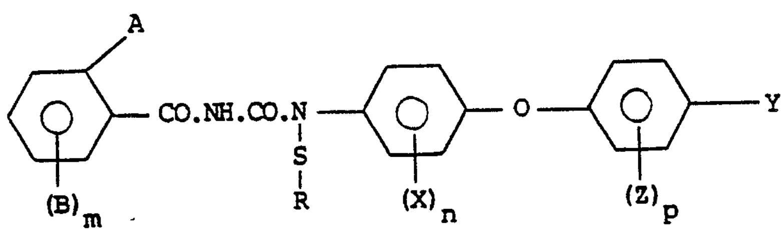

(I)

in which each of A and B independently represents a halogen atom or an alkyl group; m is 0, 1 or 2; X, Y and Z independently represents a halogen atom or a cyano, nitro, alkyl or haloalkyl group, provided that any X present is not a fluorine atom ortho to the nitrogen atom; n is 0, 1, 2, 3 or 4; p is 0, 1 or 2; and R represents a group $-CO_2R^1$, $-SO_2R^1$ or $-NR^2R^3$, in which $R^1$ represents an optionally substituted alkyl or

aryl group; $R^2$ represents an optionally substituted alkyl or aryl group; and $R^3$ represents an optionally substituted alkyl or aryl group, or a group of formula $—CO_2R^4$, $—SO_2R^4$, $—COR^4$, $—CO.CO_2R^4$, $—CO.NR^5R^6$ or $—SO_2NR^5R^6$, in which $R^4$ represents an optionally substituted alkyl or aryl group, and each of $R^5$ and $R^6$ independently represents an optionally substituted alkyl or aryl group; or $R^2$ and $R^3$ together or $R^5$ and $R^6$ together represent an optionally substituted alkylene group; in each case, the optional substituents for an alkyl or alkylene group being selected from halogen, alkoxy, alkoxycarbonyl, haloalkoxycarbonyl, alkylcarbonyl, haloalkylcarbonyl, alkylsulphonyl and haloalkylsulphonyl, and the optional substituents for an aryl group being selected from these substituents and also alkyl, haloalkyl, cyano and nitro.

2. A compound as claimed in claim 1, in which each of A and B independently represents a fluorine or chlorine atom or a methyl group, and m is 0 or 1.

3. A compound as claimed in claim 1, in which A is a fluorine atom, m is 1 and B is a fluorine or chlorine atom in the 6-position of the phenyl ring.

4. A compound as claimed in any one of claims 1 to 3, in which X represents a chlorine atom or a methyl group or a fluorine atom meta to the nitrogen atom, and n is 0 or 1.

5. A compound as claimed in any one of claims 1 to 4, in which Y represents a chlorine atom or a nitro, cyano or trifluoromethyl group, Z represents a chlorine atom or a cyano or nitro group, and p is 0 or 1.

6. A compound as claimed in claim 5, in which Y represents a trifluoromethyl group, p is 1 and Z represents a chlorine atom in a position meta to Y.

7. A compound as claimed in any one of claims 1 to 6, in which each of $R^1$, $R^2$, $R^4$, $R^5$ and $R^6$, if present, represents an unsubstituted alkyl group having up to 6 carbon atoms.

8. A compound as claimed in any one of claims 1 to 7, in which R represents a group of formula $—NR^2R^3$, and $R^3$ represents an alkyl group having up to 6 carbon atoms substituted by an alkoxycarbonyl group having up to 6 carbon atoms in the alkyl moiety, or a group of formula $—CO_2R^4$, $—SO_2R^4$, $—COR^4$, $—CO.CO_2R^4$, $—CO.NR^5R^6$ or $—SO_2NR^5R^6$.

9. A process for the preparation of a compound as claimed in any one of claims 1 to 8, which comprises reacting a compound of the general formula

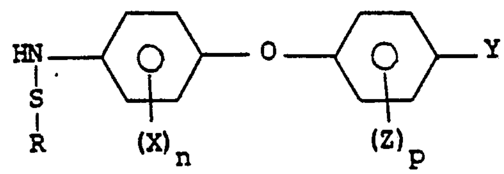

(II)

with a compound of the general formula

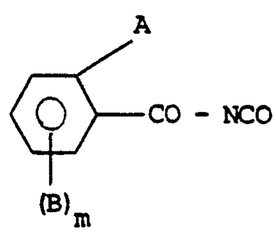

(III)

in which A, B, m, R, X, Y, Z, n and p have the meanings given in any one of claims 1 to 9.

10. A compound of the general formula II as defined in claim 9.

11. A process for the preparation of a compound as claimed in claim 10, which comprises reacting a compound of the general formula

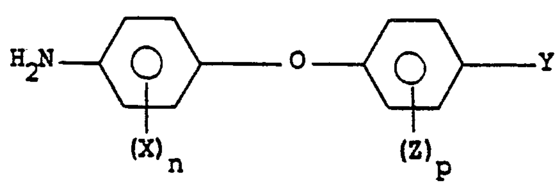

(IV)

with a compound of the general formula

Hal—S—R (V)

in which X, Y, Z, n, p and R have the meanings given above, and Hal represents a halogen atom.

12. A pesticidal composition comprising a compound as claimed in any one of claims 1 to 8, together with a carrier.

13. A composition as claimed in claim 12, which comprises at least two carriers, at least one of which is a surface-active agent.

14. A method of combating pests at a locus, which comprises applying to the locus a compound as claimed in any one of claims 1 to 8 or a composition as claimed in either claim 12 or claim 13.

15. A method according to claim 14, wherein the pests are acarids.

**Patentansprüche**

1. Verbindung der allgemeinen Formel

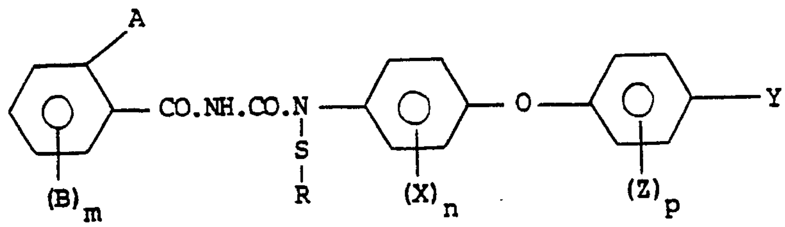

(I)

worin jeder der Reste A und B unabhängig voneinander ein Halogenatom oder eine Alkylgruppe bedeutet; m den Wert 0, 1 oder 2 aufweist; jeder der Reste X, Y und Z unabhängig voneinander ein Halogenatom oder eine Cyanogruppe, Nitrogruppe, Alkylgruppe oder Halogenalkylgruppe darstellt, mit der Maßgabe, daß jeder allenfalls vorliegende Rest X nicht ein zum Stickstoffatom orthoständiges Fluoratom bedeutet; n den Wert 0, 1, 2, 3 oder 4 aufweist; p den Wert 0, 1 oder 2 hat; und R eine Gruppe $—CO_2R^1$, $—SO_2R^1$ oder $—NR^2R^3$ darstellt, worin $R^1$ eine gegebenenfalls substituierte Alkyl- oder Arylgruppe bedeutet; $R^2$ eine gegebenenfalls substituierte Alkyl- oder Arylgruppe darstellt; und $R^3$ eine gegebenenfalls substituierte Alkyl- oder Arylgruppe bedeutet oder eine Gruppe der Formel $—CO_2R^4$, $—SO_2R^4$, $—COR^4$, $—CO.CO_2R^4$, $—CO.NR^5R^6$ oder $—SO_2NR_5R^6$ darstellt, worin $R^4$ eine gegebenenfalls substituierte Alkyl- oder Arylgruppe bedeutet und jeder der Reste $R^5$ und $R^6$ unabhängig voneinander eine gegebenenfalls substituierte Alkyl- oder Arylgruppe bezeichnet; oder $R^2$ und $R^3$ gemeinsam oder $R^5$ und $R^6$ gemeinsam eine gegebenenfalls substituierte Alkylengruppe bedeuten; wobei in jedem Falle die fakultativen Substituenten für eine Alkyl- oder Alkylengruppe ausgewählt sind unter Halogen, Alkoxy, Alkoxycarbonyl, Halogenalkoxycarbonyl, Alkylcarbonyl, Halogenalkylcarbonyl, Alkylsulfonyl und Halogenalkylsulfonyl, und die fakultativen Substituenten für eine Arylgruppe ausgewählt sind unter diesen Substituenten sowie unter Alkyl, Halogenalkyl, Cyano und Nitro.

2. Verbindung nach Anspruch 1, worin jeder der Reste A und B unabhängig voneinander ein Fluor- oder Chloratom oder eine Methylgruppe darstellt und m den Wert 0 oder 1 aufweist.

3. Verbindung nach Anspruch 1, worin A ein Fluoratom ist, n den Wert 1 hat und B ein Fluor- oder Chloratom in der 6-Stellung des Phenylringes ist.

4. Verbindung nach einem der Ansprüche 1 bis 3, worin X ein Chloratom oder eine Methylgruppe oder ein zum Stickstoffatom meta-ständiges Fluoratom darstellt und n den Wert 0 oder 1 aufweist.

5. Verbindung nach einem der Ansprüche 1 bis 4, worin Y ein Chloratom oder eine Nitro-, Cyano- oder Trifluormethylgruppe bedeutet, Z ein Chloratom oder eine Cyano- oder Nitrogruppe darstellt und p den Wert 0 oder 1 aufweist.

6. Verbindung nach Anspruch 5, worin Y eine Trifluormethylgruppe darstellt, p den Wert 1 hat und Z ein zu Y meta-ständiges Chloratom bedeutet.

7. Verbindung nach einem der Ansprüche 1 bis 6, worin jeder der Reste $R^1$, $R^2$, $R^4$, $R^5$ und $R^6$, soferne vorhanden, eine unsubstituierte Alkylgruppe mit bis zu 6 Kohlenstoffatomen bedeutet.

8. Verbindung nach einem der Ansprüche 1 bis 7, worin R eine Gruppe der Formel $—NR^2R^3$ bedeutet und $R^3$ eine Alkylgruppe mit bis zu 6 Kohlenstoffatomen, die durch eine Alkoxycarbonylgruppe mit bis zu 6 Kohlenstoffatomen im Alkylteil substituiert ist, oder eine Gruppe der Formel $—CO_2R^4$, $—SO_2R^4$, $—COR^4$, $—CO.CO_2R^4$, $—CO.NR^5R^6$ oder $—SO_2NR^5R^6$ bedeutet.

9. Verfahren zur Herstellung einer Verbindung, wie in einem der Ansprüche 1 bis 8 beansprucht, welches ein Umsetzen einer Verbindung der allgemeinen Formel

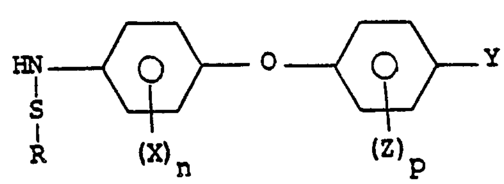

(II)

mit einer Verbindung der allgemeinen Formel

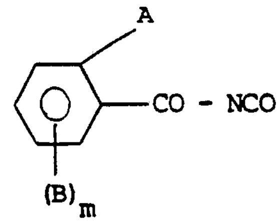 (III)

umfaßt, in welchen Formeln A, B, m, R, X, Y, Z, n und p die in einem der Ansprüche 1 bis 9 angegebenen Bedeutungen besitzen.

10. Verbindung der allgemeinen Formel II, wie in Anspruch 9 definiert.

11. Verfahren zur Herstellung einer Verbindung, wie in Anspruch 10 beansprucht, welches ein Umsetzen einer Verbindung der allgemeinen Formel

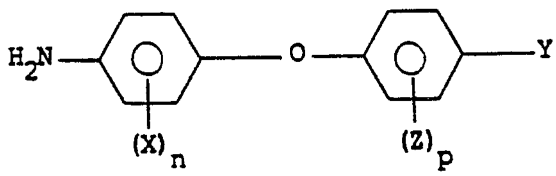 (IV)

mit einer Verbindung der allgemeinen Formel

Hal—S—R (V)

umfaßt, worin X, Y, Z, n, p und R die vorstehend angegebenen Bedeutungen und Hal ein Halogenatom bedeutet.

12. Pestizide Zusammensetzung, umfassend eine Verbindung, wie in einem der Ansprüche 1 bis 8 beansprucht, zusammen mit einem Träger.

13. Zusammensetzung, wie in Anspruch 12 beansprucht, welche wenigstens zwei Träger umfaßt, von denen wenigstens einer ein oberflächenaktives Mittel ist.

14. Verfahren zur Bekämpfung von Schädlingen an einem Ort, welches ein Aufbringen einer Verbindung, wie in einem der Ansprüche 1 bis 8 beansprucht, oder einer Zusammensetzung, wie in Anspruch 12 oder in Anspruch 13 beansprucht, auf den Ort umfaßt.

15. Verfahren nach Anspruch 14, worin die Schädlinge Akariden sind.

**Revendications**

1. Composé de formule générale

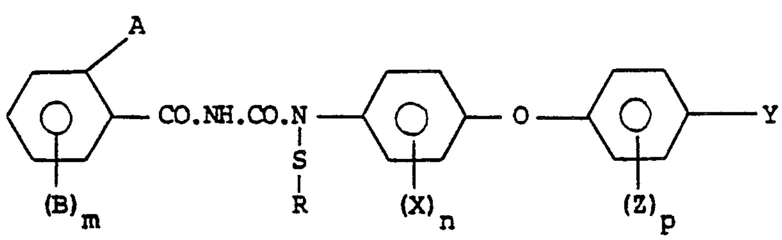 (I)

dans laquelle chacun des radicaux A et B représente indépendamment un atome d'halogène ou un groupe alkyle; m est égal à 0, 1 ou 2; chacun des radicaux X, Y et Z représente indépendamment un atome d'halogène, ou un groupe cyano, nitro, alkyle ou halogénoalkyle, à condition que tout X présent ne soit pas un atome de fluor en ortho de l'atome d'azote; n est égal à 0, 1, 2, 3 ou 4; p est égal à 0, 1, ou 2; et R représente un groupe $—CO_2R^1$, $—SO_2R^1$ ou $—NR^2R^3$, dans lequel $R^1$ représente un groupe alkyle ou aryle éventuellement substitué; $R^2$ représente un groupe alkyle ou aryle éventuellement substitué; et $R^3$ représente un groupe alkyle ou aryle éventuellement substitué ou un groupe de formule $—CO_2R^4$, $—SO_2R^4$, $—COR^4$, $—CO.CO_2R^4$, $—CO.NR^5R^6$ ou $—SO_2NR^5R^6$, dans lequel $R^4$ représente un groupe alkyle ou aryle éventuellement substitué, chacun des radicaux $R^5$ et $R^6$ représente indépendamment un groupe alkyle ou aryle éventuellement substitué; ou bien $R^2$ et $R^3$, ou $R^5$ et $R^6$, représentent ensemble un groupe alkylène éventuellement substitué; dans chaque cas, les substituants facultatifs du groupe alkyle ou alkylène étant choisis parmi les halogènes, les groupes alcoxy, alcoxycarbonyle, halogénoalcoxycarbonyle, alkylcarbonyle, halogénoalkylcarbonyle, alkylsulfonyle et halogénoalkylsulfonyle, et les substituants facultatifs du groupe aryle étant choisis parmi ces substituants et également parmi les groupes alkyle, halogénoalkyle, cyano et nitro.

2. Composé selon la revendication 1, dans lequel chacun des radicaux A et B représente indépendamment un atome de fluor ou de chlore ou un groupe méthyle, et m est nul ou égal à 1.

3. Composé selon la revendication 1, dans lequel A est un atome de fluor, m est égal à 1 et B est un atome de fluor ou de chlore en position 6 du noyau phényle.

4. Composé selon l'une quelconque des revendications 1 à 3, dans lequel X représente un atome de chlore ou un groupe méthyle ou un atome de fluor en méta de l'atome d'azote et n est nul ou égal à 1.

5. Composé selon l'une quelconque des revendications 1 à 4, dans lequel Y représente un atome de chlore ou un groupe nitro, cyano ou trifluorométhyle, Z représente un atome de chlore ou un groupe cyano ou nitro, et p est nul ou égal à 1.

6. Composé selon la revendication 5, dans lequel Y représente un groupe trifluorométhyle, p est égal à 1 et Z représente un atome de chlore en position méta par rapport à Y.

7. Composé selon l'une quelconque des revendications 1 à 6, dans lequel chacun des radicaux $R^1$, $R^2$, $R^4$, $R^5$ et $R^6$, s'il est présent, représente un groupe alkyle non substitué comportant jusqu'à 6 atomes de carbone.

8. Composé selon l'une quelconque des revendications 1 à 7, dans lequel R représente un groupe de formule $—NR^2R^3$, et $R^3$ représente un groupe alkyle comportant jusqu'à 6 atomes de carbone, substitué par un groupe alcoxycarbonyle comportant jusqu'à 6 atomes de carbone dans le groupement alkyle, ou un groupe de formule $—CO_2R^4$, $—SO_2R^4$, $—COR^4$, $—CO.CO_2R^4$, $—CO.NR^5R^6$ ou $—SO_2NR^5R^6$.

9. Procédé de préparation d'un composé selon l'une quelconque des revendications 1 à 8, qui comprend la réaction d'un composé de formule générale

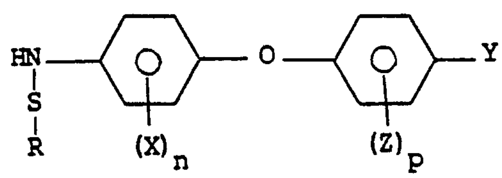

(II)

avec un composé de formule générale

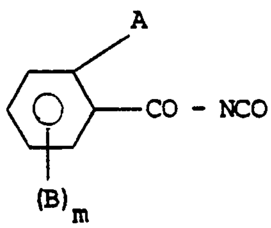

(III)

formules dans lesquelles A, B, m, R, X, Y, Z, n et p ont les significations indiquées dans l'une quelconque des revendications 1 à 8.

10. Composé de formule générale II telle que définie dans la revendication 9.

11. Procédé de préparation d'un composé selon la revendication 10, qui comprend la réaction d'un composé de formule générale

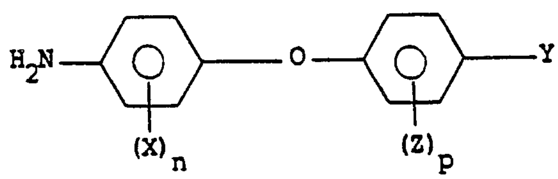

(IV)

avec un composé de formule générale

Hal—S—R (V)

formules dans lesquelles X, Y, Z, n, p et R ont les significations indiquées ci-dessus et Hal représente un atome d'halogène.

12. Composition pesticide comprenant un composé selon l'une quelconque des revendications 1 à 8, ainsi qu'un véhicule.

13. Composition selon la revendication 12, qui comprend au moins deux véhicules, dont l'un au moins est un agent tensioactif.

14. Procédé de lutte contre les parasites en un lieu, qui comprend l'application au lieu d'un composé selon l'une quelconque des revendications 1 à 8 ou d'une composition selon l'une quelconque des revendications 12 et 13.

15. Procédé selon la revendication 14, dans lequel les parasites sont des acarides.